# EUROPEAN PATENT APPLICATION

(11) **EP 4 696 304 A1**
(43) Date of publication of application: **18.02.2026**
(21) Application number: 24818436.8
(22) Date of filing: 10.05.2024
(51) Int. Cl.: A61K 9/16, A61K 47/36, A61K 47/42, A61K 47/34, A61L 27/20, A61L 27/18, A61L 27/50, A61L 27/58

(54) **PREPARATION METHOD FOR INJECTABLE PLA MICROSPHERES, AND USE THEREOF**

(30) Priority: 07.06.2023 CN 202310669766
(71) Applicant: Esunmed Biotechnology (Shenzhen) Co., Ltd, Shenzhen, Guangdong 518000 (CN); Shenzhen Esun Industrial Co., Ltd, Shenzhen, Guangdong 518000 (CN)
(72) Inventor: CHEN, Rui, Shenzhen, Guangdong 518000 (CN); ZHOU, Jiankang, Shenzhen, Guangdong 518000 (CN); XIE, Qing, Shenzhen, Guangdong 518000 (CN); YANG, Yihu, Shenzhen, Guangdong 518000 (CN)
(74) Representative: Santarelli
(86) International application number: PCT/CN2024/092247
(87) International publication number: WO 2024/250903

(57) **Abstract**

A preparation method and an application of injectable polylactic acid (PLA) microspheres are provided, which relate to the field of biomedical technologies. The preparation method of the injectable PLA microspheres include: S01, preparing a spray solution; S02, performing homogeneous emulsification on the spray solution to obtain an emulsified spray solution; and S03, drying the emulsified spray solution. The spray solution includes an oil phase and an aqueous phase, and a weight ratio of the oil phase and the aqueous phase is 1-10 : 1-10. The oil phase includes PLA and an organic solvent, and the aqueous phase includes a surfactant, macromolecular substances and deionized water. A particle size of the injectable PLA microspheres is basically below 60 µm, and the injectable PLA microspheres have a complete form, are independent of each other, do not exhibit agglomeration and have good biocompatibility, which can satisfy the requirement of the injection.

## Description

### Technical Field

The disclosure relates to the field of biomedical technologies, and more particularly to a preparation method and an application method of injectable polylactic acid (PLA) microspheres.

### Background of the Invention

In recent years, biodegradable polymer materials come into vision of people, such materials have no toxicity and rejection to a human body, and can be excreted from the human body after being gradually degraded with human metabolism. Due to its great biocompatibility and biodegradability, polylactic acid (PLA) microspheres have been used as a carrier for peptide and protein drugs, and are widely applied in numerous fields such as immunology, gene therapy, tumor therapy and ophthalmology.

Injectable microspheres have been widely used in clinical practice. For the injectable microspheres, they are gradually gradated in the human body after injection, so that a drug loaded on the microspheres is slowly released at a certain rate to maintain a plasma concentration of a lesion site is maintained, so as to extend a half-life period of the drug, and achieve long-term sustained release. For patients, a frequency of administration can be fully reduced to improve compliance. Medical injectable PLA microspheres have unlimited application possibilities with a vigorous development of consumer medical market.

The PLA microspheres can be also applied in a field of medical cosmetology. The medical cosmetology industry has developed rapidly in recent years, upstream filling materials are currently in an upgrade and iteration period, which is updated from hyaluronic acid to recycled materials such as collagen, PLA and poly-L-lactic acid (PLLA). The PLA is a polymer material synthesized by chemistry, which can achieve high purity, and has no immunogen hidden risk. A medical cosmetology filler (also referred to as dermal filler) developed based on the PLA can stimulate an immune system in the human body, and affect immune cells, so as to induce fibroblasts to up-regulate collagen secretion, and promote regeneration and repair of skin tissues.

However, in actual production, the prepared PLA microspheres are prone to agglomeration, and have poor dispersibility in water, thus, the prepared PLA microspheres are difficult to be injected. Meanwhile, a degradation rate of a single PLA microsphere is slow in an early stage, and accelerates in a later stage, which is not conducive to control of drug dosage.

### Summary of the Invention

Based on problems exist in the related art, the disclosure provides a preparation method and an application method of injectable polylactic acid (PLA) microspheres. The PLA microspheres prepared by the method of the disclosure have a complete microsphere form and a good biocompatibility, which can satisfy requirements of injection.
The disclosure is implemented through the following technical solutions.
Specifically, a preparation method of injectable PLA microspheres is provided, and the preparation method includes:
   S01, preparing a spray solution;
   S02, performing homogeneous emulsification on the spray solution to obtain an emulsified spray solution; and
   S03, drying the emulsified spray solution to obtain the injectable PLA microspheres.

In an embodiment, in the step S01, the spray solution includes an oil phase and an aqueous phase, and a weight ratio of the oil phase and the aqueous phase is 1-10 : 1-10; and the oil phase includes PLA and an organic solvent, and the aqueous phase includes a surfactant, macromolecular substances and deionized water.

In an embodiment, a molecular weight of the PLA is in a range of 50000-95000.

In an embodiment, the organic solvent is one selected from the group consisting of dichloromethane, trichloromethane and ethyl acetate.

In an embodiment, a concentration of the PLA is in a range of 20-60 grams per liter (g/L).

In an embodiment, the surfactant is one selected from the group consisting of sodium dodecylbenzene sulfonate, polysorbate 80 (also referred to as TWEEN^{®} 80), and polysorbate 20 (also referred to as TWEEN^{®} 20).

The macromolecular substances are a mixture of sodium alginate, whey protein and β-glucose, and a weight ratio of the sodium alginate : the whey protein : the β-glucose is 1-6 : 1-3 : 1-5.

In an embodiment, a concentration of the surfactant is in a range of 5-10 g/L, and a concentration of the macromolecular substances is in a range of 0.5-1 g/L.

In an embodiment, in the step S02, a temperature of the homogeneous emulsification for the spray solution is 50 Celsius degrees (°C), a rotational speed of a homogenizer is in a range of 3000-5000 revolutions per minute (rpm), and a time of the homogeneous emulsification for the spray solution is in a range of 5-10 minutes (min).

In an embodiment, in the step S03, process conditions of the drying the emulsified spray solution are as follows. An input rate of the emulsified spray solution is in a range of 4-20 milliliters per minute (mL/min), a pressure of compressed air is in a range of 0.3-0.5 megapascals (MPa), an air inlet temperature is 160°C, an air outlet temperature is 80°C, and carrier gas is inert gas or air. The PLA microspheres are collected in a cyclone separator, and are dried under reduced pressure below 60°C.

In an embodiment, an application method of the injectable PLA microspheres is provided, and the application method includes:
preparing drug sustained-release microspheres by applying the injectable PLA microspheres; where weight of the drug sustained-release microspheres is in a range of 0.1-10% of weight of the PLA microspheres.

Beneficial effects of the disclosure are as follows.
(1) The PLA microspheres prepared by a spray drying method adopted by the disclosure is prone to control particle sizes and forms of the microspheres, have a high production rate and can be mass-produced. The particle size of the injectable PLA microspheres is basically below 60 microns (µm), and the PLA microspheres have complete forms, are independent of each other, do not exhibit agglomeration and have good biocompatibility, which can satisfy the requirement of the injection. The PLA microspheres are dispersed in water, and show a good dispersibility after standing, and the PLA microspheres can be evenly dispersed in water to form even dispersion liquid, instead of floating on an upper layer or depositing on a lower layer of the water.
(2) A degradation weight loss rate of the PLA microspheres prepared in the disclosure keeps a constant rate, which is suitable for the preparation of drug sustained-release microspheres, so as to avoid problems that traditional single PLA microspheres have slow degradation in the early stage, slow drug release, rapid degradation in the later stage and uncontrollable drug dosage caused by rapid drug release.

### Brief Description of the Drawings

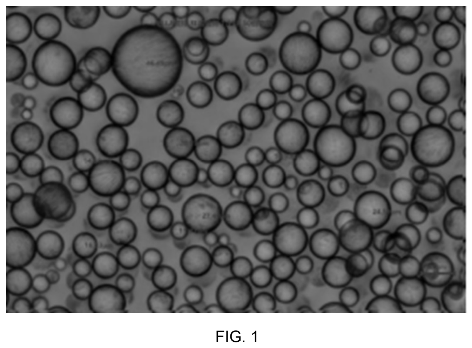

Drawing is used to provide a further description of the disclosure, and is constituted a part of the specification, the drawing is combined with embodiments of the disclosure to describe the disclosure, and the drawing does not limit the disclosure.

Figure illustrates a microscopic diagram of injectable polylactic acid (PLA) microspheres according to an embodiment 1 of the disclosure.

### Detailed Description of Embodiments

Technical solutions of the disclosure are further elaborated in conjunction with embodiments below, however, a scope of protection of the disclosure is not limited by the following embodiments.

In the embodiments and comparative embodiments of the disclosure, a molecular weight of polylactic acid (PLA) is 5000.

### Embodiment 1

Injectable PLA microspheres are prepared by the following steps S01-S03.

In step S01, a spray solution is prepared.

The spray solution includes an oil phase and an aqueous phase, and a weight ratio of the oil phase and the aqueous phase is 5 : 3.

The oil phase includes PLA and dichloromethane, and a concentration of the PLA is 40 grams per liter (g/L).

The aqueous phase includes polysorbate 20, macromolecular substances and deionized water. A concentration of the polysorbate 20 is 5 g/L, and a concentration of the macromolecular substances is 0.5 g/L.

The macromolecular substances are a mixture of sodium alginate, whey protein and β-glucose, and a weight ratio of the sodium alginate : the whey protein : the β-glucose is 2 : 1 : 1.

In step S02, homogeneous emulsification is performed on the spray solution to obtain an emulsified spray solution.

A temperature of the homogeneous emulsification for the spray solution is 50 Celsius degrees (°C), a rotational speed of a homogenizer is 4000 revolutions per minute (rpm), and a time of the homogeneous emulsification for the spray solution is 10 minutes (min).

In step S03, the emulsified spray solution is dried to obtain the PLA microspheres.

An input rate of the emulsified spray solution is 10 milliliters per minute (mL/min), a pressure of compressed air is 0.5 megapascals (MPa), an air inlet temperature is 160°C, an air outlet temperature is 80°C, and carrier gas is air. The PLA microspheres are collected in a cyclone separator, and are dried under reduced pressure below 60°C.

The PLA microspheres of the embodiment 1 is dissolved in water, after mixing, the PLA microspheres are dispersed in the water to obtain dispersion liquid. Water in the dispersion liquid is dried to obtain dried PLA microspheres, the dried PLA microspheres are observed by using a scanning electron microscope, and a result is shown in Figure. Figure illustrates a microscopic diagram of the injectable PLA microspheres according to the embodiment 1 of the disclosure. It can be seen from Figure, the PLA microspheres have complete forms, are independent of each other, do not exhibit agglomeration and have good dispersibility.

### Embodiment 2

Injectable PLA microspheres are prepared by the following steps S01-S03.

In step S01, a spray solution is prepared.

The spray solution includes an oil phase and an aqueous phase, and a weight ratio of the oil phase and the aqueous phase is 3 : 5.

The oil phase includes PLA and dichloromethane, and a concentration of the PLA is 50 g/L.

The aqueous phase includes sodium dodecylbenzene sulfonate, macromolecular substances and deionized water. A concentration of the sodium dodecylbenzene sulfonate is 5 g/L, and a concentration of the macromolecular substances is 0.5 g/L.

The macromolecular substances are a mixture of sodium alginate, whey protein and β-glucose, and a weight ratio of the sodium alginate : the whey protein : the β-glucose is 3 : 1 : 2.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Embodiment 3

Injectable PLA microspheres are prepared by the following steps S01-S03.

In step S01, a spray solution is prepared.

The spray solution includes an oil phase and an aqueous phase, and a weight ratio of the oil phase and the aqueous phase is 5 : 5.

The oil phase includes PLA and dichloromethane, and a concentration of the PLA is 40 g/L.

The aqueous phase includes polysorbate 80, macromolecular substances and deionized water. A concentration of the polysorbate 80 is 5 g/L, and a concentration of the macromolecular substances is 0.5 g/L.

The macromolecular substances are a mixture of sodium alginate, whey protein and β-glucose, and a weight ratio of the sodium alginate : the whey protein : the β-glucose is 6 : 3 : 5.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Comparative embodiment 1

Injectable PLA microspheres are prepared by the following steps S01-S03.

In step S01, a spray solution is prepared.

The spray solution includes PLA and dichloromethane, and a concentration of the PLA is 40 g/L.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Comparative embodiment 2

Injectable PLA microspheres are prepared by the following steps S01-S03.

In step S01, a spray solution is prepared.

The spray solution includes an oil phase and an aqueous phase, and a weight ratio of the oil phase and the aqueous phase is 5 : 3.

The oil phase includes PLA and dichloromethane, and a concentration of the PLA is 40 g/L.

The aqueous phase includes polysorbate 20 and deionized water, and a concentration of the polysorbate 20 is 5 g/L.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Comparative embodiment 3

Injectable PLA microspheres are prepared by the following steps S01-S03.

In step S01, a spray solution is prepared.

The spray solution includes an oil phase and an aqueous phase, and a weight ratio of the oil phase and the aqueous phase is 5 : 3.

The oil phase includes PLA and dichloromethane, and a concentration of the PLA is 40 g/L.

The aqueous phase includes polysorbate 20, macromolecular substances and deionized water. A concentration of the polysorbate 20 is 5 g/L, and a concentration of the macromolecular substances is 0.5 g/L.

The macromolecular substances are a mixture of sodium alginate and β-glucose, and a weight ratio of the sodium alginate and the β-glucose is 2 : 1.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Comparative embodiment 4

Injectable PLA microspheres are prepared by the following steps S01-S03.

In step S01, a spray solution is prepared.

The spray solution includes an oil phase and an aqueous phase, and a weight ratio of the oil phase and the aqueous phase is 5 : 3.

The oil phase includes PLA and dichloromethane, and a concentration of the PLA is 40 g/L.

The aqueous phase includes polysorbate 20, macromolecular substances and deionized water. A concentration of the polysorbate 20 is 5 g/L, and a concentration of the macromolecular substances is 0.5 g/L.

The macromolecular substances are a mixture of whey protein and β-glucose, and a weight ratio of the whey protein and the β-glucose is 1 : 1.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Comparative embodiment 5

Injectable PLA microspheres are prepared by the following steps S01-S03.

In step S01, a spray solution is prepared.

The spray solution includes an oil phase and an aqueous phase, and a weight ratio of the oil phase and the aqueous phase is 5 : 3.

The oil phase includes PLA and dichloromethane, and a concentration of the PLA is 40 g/L.

The aqueous phase includes the polysorbate 20, macromolecular substances and deionized water. A concentration of the polysorbate 20 is 5 g/L, and a concentration of the macromolecular substances is 0.5 g/L.

The macromolecular substances are a mixture of sodium alginate and whey protein, and a weight ratio of the sodium alginate and the whey protein is 2 : 1.

In step S02, homogeneous emulsification is performed on the spray solution to obtain an emulsified spray solution.

A temperature of the homogeneous emulsification for the spray solution is 50°C, a rotational speed of the homogenizer is 4000 rpm, and a time of the homogeneous emulsification for the spray solution is 10 min.

In step S03, the emulsified spray solution is dried to obtain the PLA microspheres.

An input rate of the emulsified spray solution is 10 mL/min, a pressure of the compressed air is 0.5 MPa, an air inlet temperature is 160°C, an air outlet temperature is 80°C, and the carrier gas is air. The PLA microspheres are collected in the cyclone separator, and are dried under reduced pressure below 60°C.

### Experimental embodiment 1

Comparison of weight-loss rate of degradation of the PLA microspheres is as follows.

The PLA microspheres of embodiments 1-3 and comparative embodiments 1-5 are respectively added into buffer solutions with a potential of hydrogen (pH) of 7.4 to obtain mixtures, and a degradation experiment is performed on the mixtures in a shaker with a temperature of 37°C. The mixtures are taken out at regular intervals, and are respectively centrifuged, cleaned and dried to obtain residual microspheres. Weight of the residual microspheres is weighed, and weighed results are shown in Table 1.

**Table 1**

| Groups | First month | Second month | Third month | Fourth month | Fifth month | Sixth month |
|---|---|---|---|---|---|---|
| Embodiment 1 | 92.6 | 85.4 | 61.3 | 43.5 | 33.1 | 21.8 |
| Embodiment 2 | 93.4 | 85.8 | 60.5 | 42.0 | 34.9 | 23.7 |
| Embodiment 3 | 91.7 | 84.2 | 62.3 | 44.1 | 33.7 | 21.0 |
| Comparative embodiment 1 | 95.1 | 92.9 | 90.2 | 86.7 | 66.3 | 43.2 |
| Comparative embodiment 2 | 93.4 | 91.7 | 88.5 | 84.1 | 63.0 | 39.5 |
| Comparative embodiment 3 | 92.5 | 88.4 | 70.9 | 59.4 | 35.8 | 21.6 |
| Comparative embodiment 4 | 93.2 | 86.9 | 71.8 | 55.7 | 34.6 | 19.5 |
| Comparative embodiment 5 | 92.9 | 87.4 | 72.5 | 58.4 | 36.9 | 20.8 |

Values in Table 1 are weight percentages of the residual microspheres. It can be seen from data in Table 1, a degradation rate of the microspheres of the comparative embodiment 1 is slow in the early stage, the microspheres of the comparative embodiment 1 are only degraded by 10% in the third month, and the degradation rate of the microspheres significantly increase from the fourth month to the sixth month. Therefore, it is difficult to control the drug release amount when the microspheres of the comparative embodiment 1 are used to prepare the drug sustained-release microspheres. Similarly, the degradation rate is slow in the early stage and is significantly increased in the later stage in the comparative embodiment 2. In the comparative embodiments 4 and the comparative embodiment 5, the macromolecular substances are added, and the macromolecular substances can improve the degradation rate of the microspheres in the early stage, but the degradation rate in the early and later stages is not kept at a stable level. In the embodiments 1-3 of the disclosure, three kinds of macromolecular substances are mixed to prepare the PLA microspheres, the obtained microspheres can keep a stable degradation rate, which solves the unstable situation that less degradation in the early stage and more degradation in the later stage, and can ensure a stable drug release rate when the microspheres of the embodiments 1-3 are used for preparing drug sustained-release microspheres.

### Experimental embodiment 2

Absorbability comparison of the PLA microspheres is as follows.

A disposable syringe with a volume of 1 milliliter (mL) equipped with a needle with an inner diameter of 0.5 millimeters (mm) and a length of 19.7 mm (i.e., a commonly used syringe gun head with a volume of 1 mL) is used as an injection rate detection device, and 1 mL of microsphere dispersion liquid (i.e., a dry weight of the microspheres is 0.1 g, and is recorded as MO) is used as an experimental sample. 0.1 g of the microspheres of the embodiments 1-3 and the comparative embodiments 1-5 are respectively weighed and dispersed in 1 mL of distilled water to obtain mixtures, and then placed into glass bottles, and microsphere dispersion liquids are obtained after rapid mixing. 1 mL of the microsphere dispersion liquids are respectively sucked by using the disposable syringe with a volume of 1 mL to obtain experimental samples. The experimental samples are respectively frozen and dried, and then the microspheres in the disposable syringes are respectively sucked. A percentage of weight (i.e., M1) of the microspheres in each disposable syringe to total weight (i.e., MO) of the microspheres in each experimental sample is taken as an absorbability rate of the microspheres with a calculation formula of (M1/MO) × 100%. Specific results are shown in Table 2.

**Table 2**

| Groups | Absorbability rate % |
|---|---|
| Embodiment 1 | 89 |
| Embodiment 2 | 90 |
| Embodiment 3 | 86 |
| Comparative embodiment 1 | 35 |
| Comparative embodiment 2 | 67 |
| Comparative embodiment 3 | 83 |
| Comparative embodiment 4 | 81 |
| Comparative embodiment 5 | 85 |

It can be seen from the result shown in Table 2, absorbability rates of the microspheres prepared in the embodiments 1-3 are much higher than that in the comparative embodiments 1 and 2. In the comparative embodiment 1, a single PLA is used to prepare the microspheres, and the absorbability rate is only 35%. In the comparative embodiment 2, the surfactant is added to prepare the microspheres, and the absorbability rate is nearly doubled. In the embodiments 1-3 and the comparative embodiments 3-4, the macromolecular substances are added to prepare the microspheres, and the absorbability rate is significantly increased.

### Experimental embodiment 3

Comparison of protein adsorption of the PLA microspheres is as follows.

When a biomaterial is in contact with the physiological environment, it needs good cell adhesion to avoid rejection in the body and serious side effects. An important point in cell adhesion is that a large number of proteins adhere to cell surface, and the protein adsorption is an important driving force of the cell adhesion.

A concentration of bovine serum albumin (BSA) is taken as an abscissa, and an absorbance of the BSA is taken as an ordinate, to draw a BSA growth curve. BAS solution is prepared, and an absorbance of the BSA solution is measured. In each of the embodiments 1-3 and the comparative embodiments 1-5, the BSA solution is used to incubate the PLA microspheres to obtain an incubated solution. After incubating for 5 hours (h), an absorbance of the incubated solution is measured, a concentration of the incubated solution is calculated, and a concentration difference before and after incubation indicates that the BSA is adsorbed by the PLA microspheres. Specific absorption results are shown in Table 3.

**Table 3**

| Groups | Adsorption capacity of BSA (microgram per gram abbreviated as µg/g) |
|---|---|
| Embodiment 1 | 3600 |
| Embodiment 2 | 3200 |
| Embodiment 3 | 4000 |
| Comparative embodiment 1 | 500 |
| Comparative embodiment 2 | 1200 |
| Comparative embodiment 3 | 2800 |
| Comparative embodiment 4 | 3000 |
| Comparative embodiment 5 | 2800 |

It can be seen from the result in Table 3, the adsorption capacity of the BSA of the microspheres in the comparative embodiment 1 only prepared by the PLA is 500 µg/g. It should be noted that the higher the adsorption capacity of the BSA means the better protein adsorption. In the comparative embodiment 2, the surfactant is added to prepared the microspheres, which improves the protein adsorption of the microspheres. In the comparative embodiments 3-4, the macromolecular substances are added to prepare the microspheres, which significantly improves the protein adsorption of the microspheres. In the embodiments 1-3, the adsorption capacity of the BSA can achieve 3200 µg/g, which shows that the PLA microspheres prepared by the method of the disclosure have excellent cell adhesion, and the PLA microspheres can be used to prepare the drug sustained-release microspheres, to be thereby applied in injection.

Finally, it should be noted that: the above embodiments merely show some of the embodiments of the disclosure, and are not used to limit the disclosure. For those skilled in the art, any modifications, equivalent substitutions and improvements made without departing from a concept of the disclosure shall be included in the scope of protection of the disclosure. Therefore, the scope of protection of the disclosure shall be based on claims.

## Claims

1. A preparation method of injectable polylactic acid (PLA) microspheres, comprising:
S01, preparing a spray solution;
S02, performing homogeneous emulsification on the spray solution to obtain an emulsified spray solution; and
S03, drying the emulsified spray solution to obtain the injectable PLA microspheres;
wherein in the step S01, the spray solution comprises an oil phase and an aqueous phase, and a weight ratio of the oil phase and the aqueous phase is 1-10 : 1-10; and the oil phase comprises PLA and an organic solvent, and the aqueous phase comprises a surfactant,
macromolecular substances and deionized water;
in the oil phase, a molecular weight of the PLA is in a range of 60000-95000;
a concentration of the PLA is in a range of 20-60 g/L;
the organic solvent is one selected from the group consisting of dichloromethane, trichloromethane, and ethyl acetate;
in the aqueous phase, the surfactant is one selected from the group consisting of sodium dodecylbenzene sulfonate, polysorbate 80, and polysorbate 20;
the macromolecular substances are a mixture of sodium alginate, whey protein and β-glucose, and a weight ratio of the sodium alginate : the whey protein : the β-glucose mixture is 1-6 : 1-3 : 1-5; and
a concentration of the surfactant is in a range of 5-10 g/L, and a concentration of the macromolecular substances is in a range of 0.5-1 g/L.

2. The preparation method of the injectable PLA microspheres as claimed in claim 1, wherein in the step S02, a temperature of the homogeneous emulsification for the spray solution is 50 °C, a rotational speed of a homogenizer is in a range of 3000-5000 rpm, and a time of the homogeneous emulsification for the spray solution is in a range of 5-10 minutes.

3. The preparation method of the injectable PLA microspheres as claimed in claim 1, wherein in the step S03, process conditions of the drying the emulsified spray solution comprise: an input rate of the emulsified spray solution being in a range of 4-20 mL/min, a pressure of compressed air being in a range of 0.3-0.5 MPa, an air inlet temperature being 160°C, an air outlet temperature being 80°C, and carrier gas being inert gas or air; wherein the PLA microspheres are collected in a cyclone separator, and are dried under reduced pressure below 60°C.

4. Application of the injectable PLA microspheres as claimed in any one of claims 1-3 in preparation of drug sustained-release microspheres; wherein weight of the drug sustained-release microspheres is in a range of 0.1-10% of weight of the PLA microspheres.
